(19) Europäisches Patentamt
European Patent Office
Office européen des brevets

(11) Publication number: 0 333 033 A1

# (12) EUROPEAN PATENT APPLICATION

(21) Application number: 89104189.9

(22) Date of filing: 09.03.89

(51) Int. Cl.4: C12N 15/00 , C12N 9/00 , A01N 63/00 , //(C12N15/00, C12R1:55)

(30) Priority: 09.03.88 JP 53553/88
01.04.88 JP 78095/88

(43) Date of publication of application: 20.09.89 Bulletin 89/38

(84) Designated Contracting States: BE DE FR GB NL

(71) Applicant: MEIJI SEIKA KAISHA LTD.
4-16 Kyobashi 2-chome
Chuo-ku Tokyo 104(JP)

(72) Inventor: Kumada, Yoichi c/o Meiji Seika Kaisha, Ltd.
Central Laboratories 760, Morooka-cho
Kohoku-ku
Yokohama-shi Kanagawa(JP)
Inventor: Takano, Eriko c/o Meiji Seika Kaisha, Ltd.
Central Laboratories 760, Morooka-cho
Kohoku-ku
Yokohama-shi Kanagawa(JP)
Inventor: Nagaoka, Kozo c/o Meiji Seika Kaisha, Ltd.
Central Laboratories 760, Morooka-cho
Kohoku-ku
Yokohama-shi Kanagawa(JP)
Inventor: Baba, Jun c/o Meiji Seika Kaisha, Ltd.
Pharmac. Dev. Lab. 580, Horikawa-cho
Saiwai-ku
Kawasaki-shi Kanagawa(JP)

(74) Representative: WILHELMS, KILIAN & PARTNER Patentanwälte
Eduard-Schmid-Strasse 2
D-8000 München 90(DE)

## (54) Glutamine synthesis gene and glutamine synthetase.

(57) A glutamine synthesis gene and a glutamine synthetase encoded by the gene resistant to glutamine synthetase inhibitors, which are useful for creating plant species resistant to glutamine synthetase inhibitor herbicides or breeding microorganisms capable of producing such herbicides.

EP 0 333 033 A1

# GLUTAMINE SYNTHESIS GENE AND GLUTAMINE SYNTHETASE

## FIELD OF THE INVENTION

This invention relates to a glutamine synthesis gene and glutamine synthetases as well as use thereof.

## BACKGROUND OF THE INVENTION

The prior art technology for rendering organisms resistant to the herbicides L-phosphinothricin and bialaphos includes two methods: (1) one method comprises subjecting a target organism (e.g. microorganism, plant) to mutation treatment to thereby render it resistant and (2) the other comprises cloning the gene (bar) coding for an enzyme that promotes acetylation of the amino group of L-phosphinothricin, followed by introducing the cloned gene into said organism. As an example of method (2), there may be mentioned the use of the Bar gene cloned from Streptomyces hygroscopicus SF-1293 (FERM BP-130 or ATCC-21705) (cf. EP-A-0173327) which is a bialaphos-producing strain. This gene codes for an enzyme (L-phosphinothricin acetyltransferase, PAT) catalyzing acetylation of the amino group of L-phosphinothricin, which is a constituent element of bialaphos.

The method (1) is disadvantageous in that the mechanism of resistance acquirement cannot be specified and that some other mutation having nothing to do with resistance may possibly be introduced simultaneously.

The method (2) is disadvantageous in that resistance cannot be developed to other glutamine synthetase inhibitors than L-phosphinothricin and bialaphos since said gene leads to resistance expression via specific inactivation (modification) of both substances by N-acetylation. Therefore, said gene cannot be a resistance factor against glutamine synthetase inhibitors in general.

Furthermore, it is a problem that introduction of a specific character into living organisms by a plasmid or the like possibly results in excessive production of the DNA introduced and the protein originating therefrom and, as a result, in production of a secondary adverse effect. In particular when the character to be introduced is dependent on the gene dosage of the DNA introduced and on the overproduction of the protein (enzyme) encoded by the DNA introduced, the adverse effect resulting therefrom cannot be avoided.

This problem is also encountered in creating organisms resistant to glutamine synthetase inhibitors by the introduction (gene dosage) of a multicopy glutamine synthetase gene. For example, in microbial production of the herbicide L-phosphinothricin or bialaphos, which is a glutamine synthetase inhibitor, introduction into the producer microorganism of a known glutamine synthesis gene (cf. J. Biol. Chem., 261, 10587-10591, 1986; Nature, 306, 337-342, 1983; Mol. Gen. Genet., 203, 221-229, 1986) as inserted in a multicopy vector plasmid indeed results in increased resistance but not always in increased productivity. The primary cause is that the metabolic balance in the microbial cell cannot be kept due to unnecessarily excessive production of the DNA (plasmid) introduced and the protein (glutamine synthetase) originating therefrom.

Accordingly, the advent of a resistance factor having wider applicability and higher productivity has been awaited.

## SUMMARY OF THE INVENTION

As a result of development works made by the present inventors in an attempt to establish a technique of rendering organisms resistant by amplifying the glutamine synthesis gene, a novel glutamine synthesis gene (gln-hR) for a glutamine synthetase resistant to glutamine synthetase inhibitors has been cloned and it has been found that the protein (glutamine synthetase) originating from said gene is a novel glutamine synthet ase of the type very hardly suspectible to inhibition by glutamine synthetase inhibitors.

## BRIEF DESCRIPTION OF THE DRAWINGS

Fig. 1 shows the restriction eyzyme maps of the plasmids pGSH5-29, pMSG3, and pMSG5.

Fig. 2 shows the restriction enzyme map of the plasmid pMSG9.

Fig. 3 shows the inhibitions of the glutamine synthetases GS-hR and GS-hS by L-phosphinothricin as comparatively determined by the forward reaction method.

Fig. 4 shows the base sequence of the gln-hR gene and the amino acid sequence of GS-hR.

## DETAILED DESCRIPTION OF THE INVENTION

The glutamine synthesis gene according to the invention can be obtained, for example, by cloning a gene complementary to the glutamine requirement of the glutamine synthetase-deficient Escherichia coli strain YMC11 (available from E. coli Genetic Stock Center, Department of Biology, Yale University) using the whole DNA of Streptomyces hygroscopicus SF-1293 (FERM BP-130; ATCC 21705) as the donor. This cloned glutamine synthesis gene is contained in the plasmid pMSG5. Not only the cloned substantial gene but also genes equivalent thereto fall within the scope of the present invention. The term "equivalent" is used herein to include genes which are different in base sequence from said cloned gene due to degeneracy of DNA codons but are identical in genotype with said cloned gene. The term "substantial" is used herein to include the glutamine synthetase-encoding gene and those genes which take part in the expression of said gene.

It has been found that when this gene is introduced into an actinomycete, which is to serve as a host, the resulting transformants show marked resistance to bialaphos and L-phosphinothricin.

A transformant strain, Streptomyces lividans 66/pMSG5, harboring the plasmid pMSG5 that contains said gene has been deposited at the Fermentation Research Institute (Tsukuba, Japan) under the deposit number FERM BP-2302 in accordance with the Budapest Treaty.

The glutamine synthesis gene according to the invention can give to host microorganisms very strong resistance to glutamine synthetase inhibitors (e.g. L-phosphinothricin) even if the glutamine synthetase expression is of a relatively low level. It has been found that this is because actinomycetes harboring a high-copy plasmid containing said gene can specifically produce an intracellular protein of 41 kilodaltons in large amounts. This protein is a glutamine synthetase of the type hardly susceptible to inhibition by glutamine synthetase inhibitors. This enzyme has the amino acid sequence shown in Fig. 4. The gene according to the invention has been identified as the structural gene for said enzyme by finding out, in said structural gene, a base sequence corresponding to said amino acid sequence.

This enzyme is distinctly different in subunit molecular weight and in N-terminal amino acid sequence from any of the known microorganism-derived glutamine synthetases. It is also different from the glutamine synthetase (subunit molecular weight 55 kilodaltons; inhibitor-sensitive) that is normally found in Streptomyces hygroscopicus SF-1293, which is the origin of said gene. It has thus been found that said enzyme is an absolutely new enzyme. Hereinafter, the glutamine synthesis gene is referred to as gln-hR ("gln" for glutamine synthetase, "h" for St. hydroscopicus and "R" for resistance). The glutamine synthetase resistant to glutamine synthetase inhibitors is designated as GS-hR ("GS" for glutamine synthetase, "h" for St. hygroscopicus and "R" for resistance). Furthermore, the glutamine synthetase (subunit molecular weight 55 kilodaltons) normally found in Streptomyces hydroscopicus SF-1293 and sensitive to glutamine synthetase inhibitors is hereinafter called GS-hS ("GS" for glutamine synthetase, "h" for St. hygroscopicus and "S" for sensitive).

In accordance with the invention, the glutamine synthetase resistant to glutamine synthetase inhibitors can be produced in good yields and be utilized efficiently. Typically, for example, said glutamine synthetase may be utilized in:

(1) Creating plant species resistant to glutamine synthetase inhibitor herbicides;

(2) Breeding microorganisms for use in the fermentative production of the herbicides bialaphos and L-phosphinothricin; and

(3) Studying the mechanisms of action of glutamine synthetase inhibitors.

By cloning the gln-hR gene according to the invention in pIJ61 (commercially available from John Innes Culture Collection), which is a low-copy vector plasmid, followed by transduction of actinomycetes therewith, the resistance of the hosts to glutamine synthetase inhibitors can be much increased while the glutamine synthetase activity of the hosts remains not so much changed.

The glutamine synthetases GS-hR and GS-hS have the following enzymatic and physicochemical properties:

| | Glutamine synthetase | |
|---|---|---|
| | GS-hR | GS-hS |
| Molecular weight | 41000 | 55000 |
| Isoelectric point | 6.5-7.5 | 6.5-7.5 |
| Optimum pH for enzymatic activity | 6.0 | 8.0-9.0 |
| pH range within which the enzyme is stable | 3.0-8.0 | 4.5-9.0 |
| Optimum temperature for enzymatic activity | 20-30 °C | 30-40 °C |
| Temperature at which the enzymatic activity is stable | ≦ 40 °C | ≦ 50 °C |

The following examples illustrate the invention in further detail but are by no means limitative of the scope thereof.

## Example 1

### Cloning of glutamine synthesis gene

A gene complementary to the glutamine requirement of the strain YMC11 of Escherichia coli (obtained from E. coli Genetic Stock Center, Department of Biology, Yale University), which is a glutamine synthetase-deficient strain, was cloned in the following manner, using the whole DNA of Streptomyces hygroscopicus SF-1293 (FERM BP-130; ATCC 21705), which is a bialaphos producing strain, as the donor.

First, a recombinant DNA was constructed from the donor DNA and the Escherichia coli vector plasmid pUC19 (Pharmacia) by a technique commonly used in gene manipulation. Thus, 10 μg of the donor DNA pretreated with the restriction enzyme EcoRI and 2 μg of the E. coli vector plasmid pUC19 also pretreated with the restriction enzyme EcoRI were combined, subjected to treatment with phenol to deactivate the restriction enzyme, extraction with ether to remove the residual phenol and precipitation with ethanol to precipitate DNA and then ligated together in the presence of T4 DNA ligase to give, in the reaction mixture, a recombinant DNA with the EcoRI digest of the donor DNA being inserted into pUC19 at the EcoRI site in the multilinker site thereof, with a certain frequency.

Then, the reaction mixture containing said recobminant DNA was used to transform the above-mentioned strain YMC11 by the method of Mandel (Mandel, M. and High, A.: J. Mol. Biol., 53, 159, 1970) most frequently used in the transformation of Escherichia coli. Ampicillin resistant transformants capable of forming a colony on a complete medium (L-agar) containing ampicillin (100 μg/ml) were subjected to replica plating onto glutamine-free W-salt agar medium (1.05% $K_2HPO_4$, 0.45% $KH_2PO_4$, 0.005% $MgSO_4$, 0.4% glucose, 10 μg/ml thiamine, 0.2% aspartic acid, 0.2% $NH_4Cl$, 100 μg/ml ampicillin, 2% agar). Plasmids were isolated from 10 out of the transformant strains capable of forming a colony on the latter medium. They has the common structure shown in Fig. 1 (A). The strain YMC11 was retransformed with one of the plasmids, pGSH5-29; all ampicillin-resistant transformants could grow on the above-mentioned W-salt medium (glutamine was no more required).

The region from BamHI site to EcoRI site (containing the portion having activity complementary to the glutamine requirement of the strain YMC11) was excised from the plasmid in the conventional manner. Thus, the plasmid was treated with the restriction enzymes BamHI and EcoRI, the digest was fractionated by agarose gel electrophoresis and the desired fraction was extracted from the agarose gel by electroelution, precipitated with ethanol and combined with the actinomycete vector plasmid pIJ486 (commercially available from John Innes Culture Collection) preliminarily treated with the restriction enzymes BamHI and EcoRI, with phenol, with ether, and with ethanol for precipitation, and ligation was carried out in the presence of T4 DNA ligase to give, in the reaction mixture, a recombinant DNA from the BamHI-EcoRI DNA fragment (1.7 kb) containing the portion having activity complementary to the glutamine requirement of the strain YMC11 and pIJ486 with a certain frequency.

The recombinant DNA was introduced into Streptomyces lividans 66 (FERM BP-737) and thiostrepton-resistant transformants were first selected. Then, from among them, neomycin-sensitive transformants were selected, and plasmids were reextracted therefrom. The plasmid having the structure shown in Fig. 1 (B) was named pMSG3.

The BamHI-BglII fragment (containing the portion having activity complementary to glutamine requirement) was excised from pMSG3 in the same manner and inserted into pIJ702 (commercially available from John Innes Culture Collection) at the BglII site thereof. The resultant plasmid had the structure shown in Fig. 1(C) and was designated as pMSG5.

The plasmid introduction into the actinomycete (transformation), isolation of thiostrepton-resistant transformants, isolation of neomycin-sensitive transformants and plasmid extraction from actinomycetes were carried out as described by Murakami et al. (Mol. Gen. Genet., 205, 42-50, 1986). Transformation of Streptomyces coelicolor FS 10 (obtained from University of Kentucky), which is a glutamine biosynthesis-deficient strain, with pMSG3 and with pMSG5 revealed that both plasmids were complementary to the glutamine requirement of said strain.

## Example 2

### Subcloning of gln-hR gene in middle-copy plasmid pIJ61

A DNA fragment containing the gln-hR gene was subcloned in the middle-copy plasmid pIJ61 (commercially available from John Innes Culture Collection).

Thus, in the conventional manner, pMSG5 was partially digested with the restriction enzyme BglII, the digest was fractionated by agarose gel electrophoresis, and the BglII-BglII fragment (containing an internal BglII site) was extracted by electroelution. This fraction was precipitated with ethanol and combined with the actinomycete vector plasmid pIJ61 preliminarily treated with the restriction enzyme BamHI, with phenol, with either, and with ethanol for precipitation, and ligation was carried out in the presence of T4 DNA ligase to give, in the reaction mixture, a recombinant DNA, with a certain frequency. The recombinant DNA was introduced into Streptomyces lividans 66.

For the selection of recombinant-carrying transformants, thiostrepton-resistant and ribostamycin- or neomycin-sensitive transformants were selected, and plasmids were reextracted therefrom and examined for restriction enzyme cleavage pattern for structure confirmation (Fig. 2; pMSG9).

## Example 3

### Base sequence analysis of gln-hR gene

The nucleotide base sequence of gln-hR was determined according to the method as described in S. Henikoff, Gene 28 (1984) 351-359 and C. Y. Perron et al., Gene 33 (1985) 103-119.

pMSG5 was digested with the restriction enzymes EcoRI and BamHI, the digests were subjected to agarose gel electrophoresis and the 1.7 kb DNA fragment containing gln-hR gene was isolated from the gel by electroelution. This 1.7 kb DNA fragment was cloned in M13 phage single-stranded DNA and this phage DNA was treated with Exonuclease III and Mung bean nuclease to obtain various gln-hR gene deletion mutants. A single-stranded DNA of each deletion mutant (phage) was prepared and subjected to sequencing by the dideoxy method.

As a result, the DNA base sequence of gln-hR gene was determined and the amino acid sequence of GS-hR was deduced therefrom, as shown in Fig. 4.

In Fig. 4, the N-terminal amino acid deduced from nucleotide base sequence is valine while the actual GS-hR begins with the second amino acid serine. The C-terminus was determined based on the termination codon (TGA: 1148-1150). Immediately thereafter, there are two sequences each estimable as a transcription termination site (indicated by → ←; 1153-1190 and 1160-1199).

The molecular weight of GS-hR as estimated by the analysis of the gln-hR gene is 37.1 kilodaltons and the total number of amino acids (exclusive of said valine coded by initiation codon) is 337.

## Example 4

### Purification of glutamine synthetase GS-hR resistant to glutamine synthetase inhibitors

(Cultivation)

The pMSG5-carrying strain (FERM BP-2302) of *Streptomyces lividans* was inoculated into 10 ml of a liquid medium containing 2.0% starch. 1.0% peptone, 0.3% meat extract and 0.5% dipotassium phosphate, pH 7.0, and cultivated at 28° C for 24 hours with aeration and agitation to give a seed culture. The seed culture was inoculated (inoculum size: 2%) into a medium having the following composition: 7.0% glucose, 3.9% wheat germ, 2.5% soluble vegetable proteins, 0.3% monopotassium phosphate, 0.0001% cobalt chloride, pH 6.8. Cultivation was carried out at 28° C for 3 days with aeration and agitation.

Preparation of cell-free extract

A 50-ml portion of the culture broth was placed in a centrifuge tube and centrifuged at 3,000 rpm for 10 minutes. The supernatant was discarded, the cells were suspended in imidazole buffer (20 mM imidazole, 1 mM $MnCl_2$, pH 7.5) and centrifugation was carried out again. This procedure was repeated twice for washing the cells. The wet cells (20 ml) were suspended in 30 ml of the same buffer and disrupted by sonication. Centrifugation was carried out at 12,000 rpm for 20 minutes and the supernatant was dialyzed three times against 20 mM imidazole buffer (1 mM $MnCl_2$, pH 7.5) at 4° C. The dialyzate was used for the following purification.

Step 1 : Purification using Mono Q as carrier

The ion exchange chromatography column Mono Q HR 5/5 (Pharmacia) sufficiently equilibrated with the imidazole buffer mentioned above was charged with 10 ml of the above-mentioned cell-free extract. The same imidazole buffer was passed through the column for a while. Proteins adsorbed were eluted with NaCl (0 to 1 M). The fraction containing a protein having glutamine synthetase activity and showing a molecular weight of 41 kilodaltons in SDS electrophoresis was collected. Thus was obtained a crude sample of the glutamine synthetase GS-hR resistant to glutamine synthetase inhibitors. (SDS electrophoresis was carried out as described in Nature, 227, 680-685, 1970.)

Step 2 : Purification using Superose 6 as carrier

The crude sample of GS-hR obtained in the above Step 1 was applied to a column of Superose 6 HR 10/30 (Pharmacia; carrier for protein fractionation based on the gel filtration principle) which was sufficiently equilibrated in advance with imidazole buffer, the above-mentioned imidazole buffer was passed through the column, and the eluate was fractionated. The fraction containing a protein having glutamine synthetase activity and showing a molecular weight of 41 kilodaltons in SDS electrophoresis was collected. Thus was obtained a purified sample of the glutamine synthetase GS-hR resistant to glutamine synthetase inhibitors.

## Example 5

Purification of glutamine synthetase GS-hS sensitive to glutamine synthetase inhibitors

(Cultivation)

Streptomyces hygroscopicus SF-1293 (FERM BP-130 or ATCC 21705), a bialaphos-producing strain, was inoculated into 10 ml of a liquid medium containing 2.0% starch, 1.0% peptone, 0.3% meat extract and 0.05% dipotassium phosphate, pH 7.0, and cultivated at 28°C for 24 hours with aeration and agitation to give a seed culture. The seed culture was inoculated (inoculum size 2%) into a medium having the composition: 7.0% glucose, 3.9% wheat germ, 2.5% soluble vegetable proteins, 0.3% monopotassium phosphate, 0.0001% cobalt chloride, pH 6.8. Cultivation was carried out at 28°C for 3 days with aeration and agitation.

Preparation of cell-free extract

A 50-ml portion of the culture broth was placed in a centrifuge tube and centrifugated at 3,000 rpm for 10 minutes. The supernatant was discarded, the cells were suspended in imidazole buffer (20 mM imidazole, 1mM $MnCl_2$, pH 7.5), and centrifugation was again performed. This procedure was repeated twice for washing the cells. The wet cells (20 ml) were suspended in 30 ml of the same buffer and sonicated. Centrifugation was performed at 12,000 rpm for 20 minutes and the supernatant was dialyzed three times against 20 mM imidazole buffer (1 mM $MnCl_2$, pH 7.5) at 4°C. The dialyzate was used for the following purification.

Step 1 : Purification using Blue Sepharose CL-6B as carrier

Blue Sepharose CL-6B (12 g) (Pharmacia) sufficiently equilibrated with the above-mentioned imidazole buffer was added to 90 ml of the above-mentioned cell-free extract, and the mixture was stirred gently at 4°C for 24 hours and then packed into a column. The above- mentioned imidazole buffer supplemented with 1 M NaCl was passed through the column for a while and then proteins adsorbed were eluted with the NaCl-free imidazole buffer. The fraction (crude GS-hS sample) containing a protein having glutamine synthetase activity and showing a molecular weight of 55 kilodaltons in SDS electrophoresis was collected.

Step 2 : Purification by reversed phase HPLC

The crude GS-hS sample obtained in the above Step 1 (Blue Sepharose CL-6B) was further purified by reversed phase HPLC. Inertsil 300C8 (4.6 x 100 mm; Gas Chro Kogyo) was used as the reversed phase carrier, and 0.1% TFA (trifluoroacetic acid) as the mobile phase. The crude GS-hS sample was applied to the carrier column and developed at a flow rate of 0.6 ml/min. The main protein eluted at about 38 minutes (detection: 280 nm) was collected and used as a purified GS-hS sample.

## Example 6

Amino acid sequences of Gs-hR and GS-hS

Amino acid sequences of GS-hR and GS-hS from N-terminus were determined using an amino acid sequencer. Thus, the purified GS-hR sample obtained in Example 4 and the purified GS-hS sample obtained in Example 5 were each dissolved in 0.5% SDS and analyzed by means of an amino acid sequence analyzer (Applied Biosystems 470A type). The results of the analysis are shown in Table 1 in comparison with the data for known glutamine synthetases.

Table 1 N-terminal amino acid sequence comparison of GS-hR and GS-hS with known glutamine synthetases

| Glutamine synthetase | N-terminus Amino acids (1 2 3 4 5 6 7 8 9 10 11 12 13 14 15 16 17 18 19) |
|---|---|
| GS-hR | Ser Ile Lys Ala Glu Tyr Ile Trp Ile Asp Gly Thr Gln Pro Thr Ala Lys Leu Arg |
| GS-hS | Met Phe Gln Asn Ala(Asp Glu)Ala Lys Phe Ile Ala(Asp Glu) |
| E.coli(1) | Ser Ala Glu His Val Leu Thr Met Leu Asn Glu His Glu Val |
| Cyanobacterium Anabaena (blue-green alga)(2) | Met Thr Thr Pro Gln Glu Val Leu Lys Arg Ile Gln Asp Glu Lys |
| Medicago sativa (alfalfa)(3) | Met Ser Ler Leu Ser Asp Leu Ile Asn Leu Asp Leu Ser Glu Thr |

References: (1) J.Biol.Chem. Vol.261,10587-10591,1986
(2) Nature Vol.306,337-342(1983)
(3) Mol Gen Genet(1986)203:221-229

As shown in Table 1, GS-hR and GS-hS were different in N-terminal amino acid sequence from said known glutamine synthetases.

## Example 7

Bialaphos resistance and glutamine synthetase activity of gln-hR-carrying strains (actinomycetes)

Each bacterial strain specified in Table 2 was inoculated into bialaphos-containing minimum agar media (0.75% soluble starch, 0.075% $KH_2PO_4$, 0.0025% $MgSO_4$, 0.17% $NaNO_3$, 0.000025% $CoCl_2$, 1.5% agar, pH 7.0, tap water used; bialaphos concentrations: 0, 10, 30, 100, 300, 1,000, 3,000, 10,000, 15,000, 20,000 µg/ml), cultivated at 28° C for 3 days and then examined for growth.

Glutamine synthetase activity

The activity was determined at 37° C by the γ-GT method (J. Bacteriol., 129, 1001-1008, 1976). The activity values thus found are shown in Table 2 each in terms of relative activity per milligram of protein with the activity of the parent strain being taken as 100%.

Table 2

| Bioalaphos resistance and glutamine synthetase activity data for gln-hR -carrying bacterial strains | | | | | |
|---|---|---|---|---|---|
| Strain | Plasmid | Vector | Copy number | Bialaphos resistance (M.I.C.) µg/ml | Glutamine synthetase activity (%) |
| St. lividans66 | none | | | 1.0 | 100 |
| | pMSG3 | pIJ486 | high | 100 | 1S0 |
| | pMSG5 | pIJ702 | high | 1000 | 925 |
| | pMSG9 | pIJ 61 | middle | 300 | 65 |
| St.hygroscopicus | none | | | 10 | 100 |
| SF-1293(Bar-) | pMSG3 | pIJ486 | high | 1000 | 205 |
| | pMSG5 | pIJ702 | high | 15000 | 1100 |
| | pMSG9 | pIJ 61 | middle | 15000 | 110 |
| St. hygroscopicus SF-1293(bar.): cf. JP-A-63-267284 (the term "JP-A" as used herein means "an unexamined published Japanese patent application") | | | | | |

As shown in Table 2, the bialaphos resistance of each gln-hR-carrying transformant is approximately dependent on the extent of expression of glutamine synthetase activity. In particular, marked activity increase is noted with pMSG5 in which pIJ702 is the vector plasmid. Nevertheless, in some cases, bialaphos resistance expression is observable without so much increase in said activity, as in the cases of pMSG3 and pMSG9. This is presumably due to the fact that the glutamine synthetase produced as a result of introduction of the gln-hR gene is resistant to glutamine synthetase inhibitors (e.g. L-phosphinothricin), as indicated in Example 7.

## Example 8

L-Phosphinothricin resistance of glutamine synthetase GS-hR

The glutamine synthetase GS-hR purified in Example 4 and the glutamine synthetase GS-hS purified in Example 5 were compared with respect to inhibition by L-phosphinothricin by the forward reaction method (J. Bacteriol., 129, 1001-1009, 1977). The results are shown in Fig. 3.

The present invention has thus made it possible to create plant species resistant to those herbicides which have glutamine synthetase (glutamine synthesizing enzyme) inhibiting activity and to breed microorganisms participating in the production of bialaphos and L-phosphinothricin.

While the invention has been described in detail and with reference to specific embodiments thereof, it will be apparent to one skilled in the art that various changes and modifications can be made therein without departing from the spirit and scope thereof.

## Claims

1. A glutamine synthesis gene which encodes a glutamine synthetase resistant to glutamine synthetase inhibitors.

2. The glutamine synthesis gene of claim 1 which has the DNA base sequence shown in Fig. 4.

3. The glutamine synthesis gene of claim 1 in which said glutamine synthetase inhibitors are the herbicides L-phosphinothricin and bialaphos.

4. A glutamine synthetase encoded by the gene of claim 1 and resistant to glutamine synthetase inhibitors.

5. The glutamine synthetase of claim 4 which is produced by culturing the strain Streptomyces lividans 66/pMSG5 (FERM BP-2302), and recovering from the cultured cells.

6. The glutamine synthetase of claim 4 which has the following enzymatic and physicochemical properties:

1) Molecular weight: 41,000;
2) Isoelectric point: 6.5-7.5;
3) Optimum pH for enzymatic activity: 6.0;
4) pH range within whcih the enzyme is stable: 3.0-8.0;
5) Optimum temperature for enzymatic activity: 20-30 °C;
6) Temperature at which the enzymatic activity is stable: ≤ 40 °C

7. The glutamine synthetase of claim 4 which has the amino acid sequence shown in Fig. 4.

8. The glutamine synthetase of claim 4 in which said glutamine synthetase inhibitors are the herbicides L-phosphinothricin and bialaphos.

9. An organism resistant to glutamine synthetase inhibitors in which the gene (gln-hR) of claim 1 is introduced.

10. A glutamine synthetase, GS-hS, extracted from Streptomyces hygroscopicus SF1293 (FERM BP-130 or ATCC 21705) and sensitive to glutamine synthetase inhibitors.

Neu eingereicht / Newly filed
Nouvellement déposé

FIG. 1

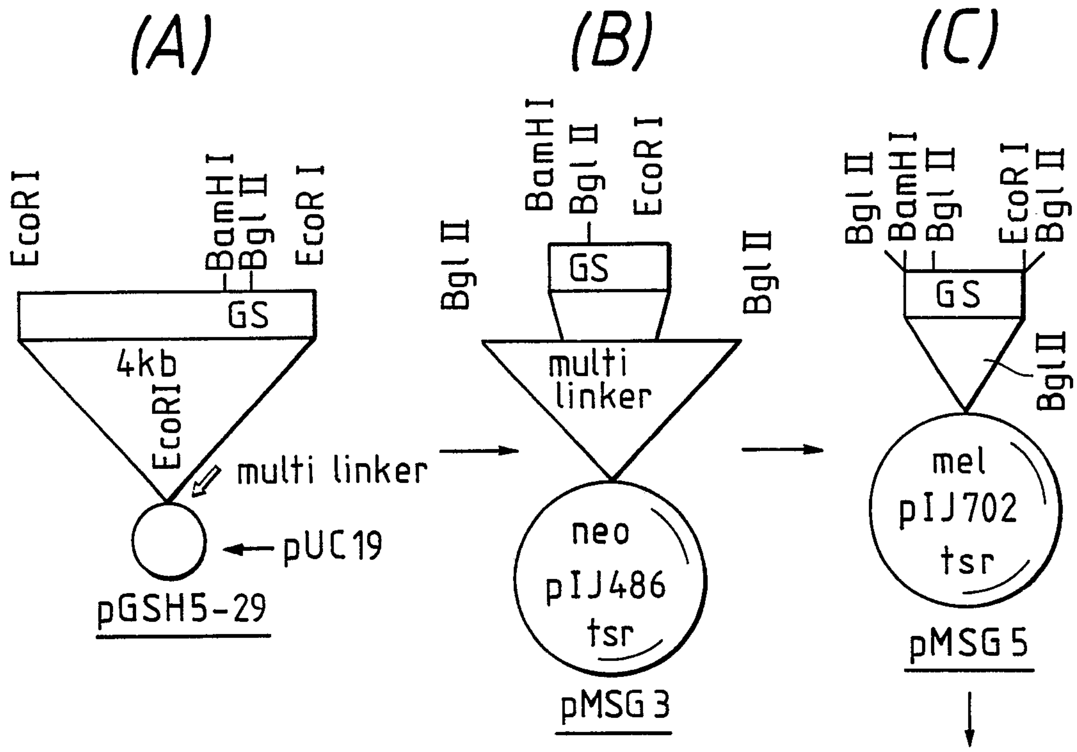


FIG. 2

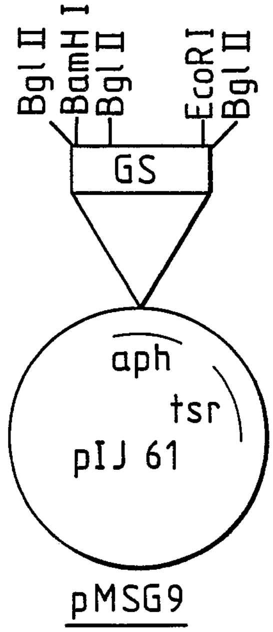

Neu eingereicht / Newly filed
Nouvellement déposé



FIG. 3

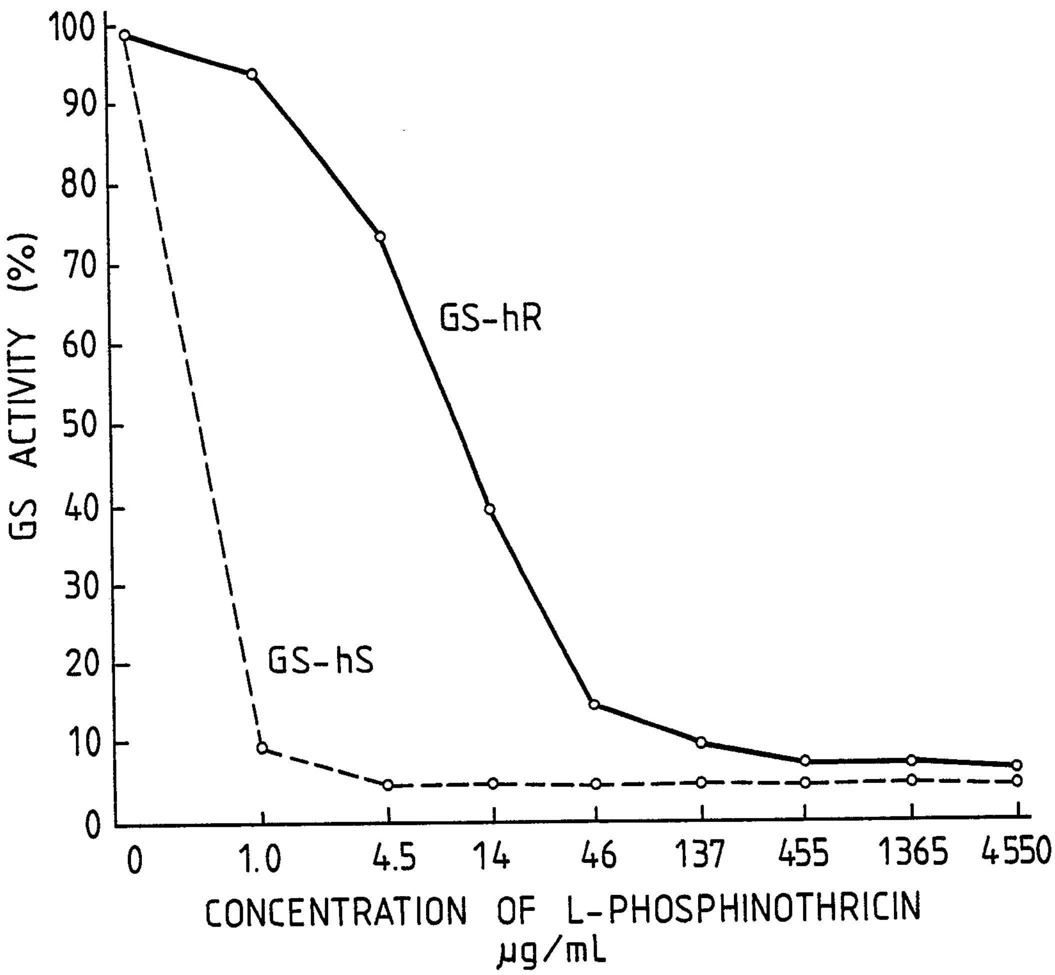

# FIG. 4

NUCLEOTIDE SEQUENCE OF gln-hR GENE AND DEDUCED AMINO ACID SEQUENCE OF GS-hR

```
                                                            60
AATTCCTTGGAAAGGGCTGGATGTCCGGCTCGGTAACCTGTGGTTCACAACGGGCAACGGA
                                                           120
CGAGAAATGGCGCGTTGCGAGAGTGCGGAGGTACGCCCGCACCGTACCCGCACCGACCGT
                                                           180
GAAGAGGACCCCGTGAGCATCAAGGCCGAGTACATCTGGATCGACGGCACGCAGCCGACC
                 ValSerIleLysAlaGluTyrIleTrpIleAspGlyThrGlnProThr
                                                           240
GCCAAGCTCCGCTCCAAGACCAAGATCCTGTCCGACGGCAGCCGGCTTCCGCGGTGGGGC
AlaLysLeuArgSerLysThrLysIleLeuSerAspGlySerArgLeuProArgTrpGly
                                                           300
TTCGACGGTTCCAGCACCAACCAGGCCGAAGGCCACGCCTCGGACCTCGTACTGGAGCCG
PheAspGlySerSerThrAsnGlnAlaGluGlyHisAlaSerAspLeuValLeuGluPro
                                                           360
GTGTTCAGCTGCCCGGACCCGATCCGCGGCGGCGACCACCTGCTGGTGCTGTGCGAGGTG
ValPheSerCysProAspProIleArgGlyGlyAspHisLeuLeuValLeuCysGluVal
                                                           420
CTGCACACCGACCTCACCCCGCACCCCTCCAACACCCGGGCGCTGCTGCGCCCGGTCGCG
LeuHisThrAspLeuThrProHisProSerAsnThrArgAlaLeuLeuArgProValAla
                                                           480
GAGCGGTTCGCCGGCCAGGAGCCGATCTTCGGCATCGAGCAGGAGTACACCTTCCTCAAG
GluArgPheAlaGlyGlnGluProIlePheGlyIleGluGlnGluTyrThrPheLeuLys
                                                           540
GGCGACCGCCCGCTCGGCTTCCCCGAGGGCGGCGGCTACCCGGCCCCGCAGGCGGACTAC
GlyAspArgProLeuGlyPheProGluGlyGlyGlyTyrProAlaProGlnAlaAspTyr
                                                           600
TACTGCGGCGTGGGCGCCATCTTCGGCCGGGAGATCGTCGAGAAGCACCTCGACCTGTGC
TyrCysGlyValGlyAlaIlePheGlyArgGluIleValGluLysHisLeuAspLeuCys
                                                           660
CTGGCGGCCGGTCTGGGCCTGTCCGGCATCAACGCCGAGGTCATGCCCGGCCAGTGGGAG
LeuAlaAlaGlyLeuGlyLeuSerGlyIleAsnAlaGluValMetProGlyGlnTrpGlu
                                                           720
TTCCAGGTCGGCGCGCTGCCGCCGCTGGAGGTCTCGGACCACATGTGGGTGGCGCGCTGG
PheGlnValGlyAlaLeuProProLeuGluValSerAspHisMetTrpValAlaArgTrp
                                                           780
CTGCTGCACCGGGTGGCGGAGGAGTTCGGCGTCACCGCGTCGCTGGACGCCAAGCCGGCC
LeuLeuHisArgValAlaGluGluPheGlyValThrAlaSerLeuAspAlaLysProAla
                                                           840
AAGGGCGACTGGAACGGCGCGGGCGCGCACACCAACTTCTCCACCCGCGCGATGCGCGAG
LysGlyAspTrpAsnGlyAlaGlyAlaHisThrAsnPheSerThrArgAlaMetArgGlu
                                                           900
GGCTACGACCCGATCATCACCGCCTGCGAGGCGCTGGGCCAGGACGACAAGCCGCTGGAG
GlyTyrAspProIleIleThrAlaCysGluAlaLeuGlyGlnAspAspLysProLeuGlu
                                                           960
CACGTCCGCCAGTACGGCACCGGCATCGAGGACCGGCTGACCGGCGCGCACGAGACCGCC
HisValArgGlnTyrGlyThrGlyIleGluAspArgLeuThrGlyAlaHisGluThrAla
                                                          1020
CCCTGGGACGCGTACTCCTACGGCGCCTCCGACCGCGGCGCCTCGGTGCGCATCCCCTGG
ProTrpAspAlaTyrSerTyrGlyAlaSerAspArgGlyAlaSerValArgIleProTrp
                                                          1080
CAGGTCGAGGTCGAGAAGAAGGGCTACATCGAGGACCGGCGCCCGAACGCCAACGTCGAC
GlnValGluValGluLysLysGlyTyrIleGluAspArgArgProAsnAlaAsnValAsp
                                                          1140
CCGTACGTGGTCACCCGGCTGATGGTGGACACCTGCTGCACGGAGCTGGCGCGGCGCGAG
ProTyrValValThrArgLeuMetValAspThrCysCysThrGluLeuAlaArgArgGlu
                                                          1200
CAGATCTGACGCCTGCGGTCACGTGGCCGTGCGGTCACGTGACCGCACGGTCGCGCGAG
GlnIle***
```

European Patent Office

# EUROPEAN SEARCH REPORT

Application number

EP 89104189.9

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | EP - A1 - 0 239 801 (THE GENERAL HOSPITAL CORPORATION) * Claims 1,2,4-7,9,11-14,19 * -- | 1,3, 8-10 | C 12 N 15/00<br>C 12 N 9/00<br>A 01 N 63/00<br>//(C 12 N 15/00<br>C 12 R 1:55) |
| A | EP - A1 - 0 240 792 (THE GENERAL HOSPITAL CORPORATION) * Claims * ---- | 1,3,4, 8,9 | |
| | | | TECHNICAL FIELDS SEARCHED (Int. Cl.4) |
| | | | C 12 N<br>A 01 N |

The present search report has been drawn up for all claims

| Place of search | Date of completion of the search | Examiner |
|---|---|---|
| VIENNA | 12-06-1989 | BÖHM |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document